(19) Europäisches Patentamt
European Patent Office
Office européen des brevets

(11) EP 4 483 904 A1

(12) **EUROPEAN PATENT APPLICATION**

(43) Date of publication:
**01.01.2025 Bulletin 2025/01**

(21) Application number: 23182286.7

(22) Date of filing: **29.06.2023**

(51) International Patent Classification (IPC):
**A61K 47/52** (2017.01)

(52) Cooperative Patent Classification (CPC):
**A61K 47/52; A61K 35/12**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Universität Wien**
**1010 Wien (AT)**

(72) Inventors:
• **ROMPEL, Annette**
**1090 Vienna (AT)**
• **NAU, Werner M.**
**28759 Bremen (DE)**
• **BARBA-BON, Andrea**
**28759 Bremen (DE)**
• **GUMEROVA, Nadiia**
**1090 Vienna (AT)**

(74) Representative: **Gassner, Birgitta et al**
**REDL Life Science Patent Attorneys**
**Donau-City-Straße 11**
**1220 Wien (AT)**

(54) **POLYOXOMETALATES AS MEMBRANE CARRIERS**

(57)    The present invention relates to a delivery molecule comprising a carrier molecule and a cargo molecule, wherein said carrier molecule is a polyoxometalate which exhibits a charge status of -2 to -6 and the cargo molecule is a hydrophilic moiety or an organic molecule and to the use of said delivery molecule in therapy.

**EP 4 483 904 A1**

## Description

### Field of the Invention

The present invention relates to the field of delivering a molecule into a cell via a carrier moiety.

### Background Art

Efficient foreign protein delivery into living cells can completely bypass transcription translation processes related to gene expression, reducing the time required for target protein synthesis from days to hours.

The design of carriers to assist the passage of bioactive therapeutic compounds through the cellular membrane presents a challenge in life sciences. Different approaches have been attempted to date, mainly focusing on the use of synthetic pores, nanoparticles and lipide formulations, cell-penetrating peptides and counterions activators. However, these systems have been deprived of their pharmaceutical application due to limitations such as endocytic entrapment, aggregation propensity, membrane-lytic activity, and related toxicity.

In WO2011/045296A a composition for delivering a peptide or a protein into a cell is disclosed. The composition comprises a polycationic agent and a polyanionic agent, wherein the polyanionic agent is an inorganic polyphosphate or a polyoxometalate. Zhang Z. et al. describe interactions between nanoparticles and cells by the development of polyoxometalates nanoparticle-peptide conjugates and investigation of their intracellular trafficking behaviors [1]. A method for transducing a protein or peptide into a cell, comprising a step of transporting the protein or peptide into the cell by using a conjugate formed by binding the protein or peptide with a polymer having a cation value of more than 2 and no more than 30,000 is disclosed in EP1316318A.

The systematic understanding of the peculiar effects that different ions exert on the biological matter can be traced back to Hofmeister, who studied their interactions with proteins. This has led to the formulation of the classical Hofmeister scale, where ions were subsequently classified as being kosmotropic if they showed salting-out properties towards proteins and as chaotropic if they displayed salting-in effects. Subsequently, these hydration effects have been generalized, the notion that the effects are a consequence of property changes of the bulk liquid has been abandoned, and, instead, direct interactions between chaotropic ions and dissolved organic matter ("chaotropic effect") have moved into the focus. Although inorganic cluster anions of the boron and polyoxometalate (POMs) type have been intensively researched for many decades, their chaotropicity has moved into the focus of attention when it was found that these large cluster ions behave as "superchaotropic" ions, that is, they exceed the chaotropic behavior of the hitherto described anions by far. Since the utilization of dodecaborates ($B_{12}X_{12}^{2-}$) as membrane carriers has been introduced [2] and their activity could be correlated with their superchaotropic nature, the exploration of this new application line to POMs is timely.

Therefore, there is still the need for a carrier molecule which may act as a new class of chaotropic membrane carriers to facilitate the transport of hydrophilic molecules across the cellular membrane.

### Summary of invention

It is the object of the present invention to provide a delivery molecule which facilitates the transport of molecules of interest across the cellular membrane. The object is solved by the subject matter of the present invention. The present invention relates to a delivery molecule which enables a cargo molecule to be translocated into cells, and a method for transducing the cargo molecule into the cells using said delivery molecule.

According to the invention, there is provided a delivery molecule comprising a polyoxometalate as carrier molecule and a cargo molecule, wherein said polyoxometalate exhibits a charge status of -2 to -6. The cargo molecule may be a hydrophilic moiety or an organic molecule. The carrier molecule and the cargo molecule are preferably linked to each other without a linker molecule.

According to one embodiment of the invention the delivery molecule as described herein comprises a poly-oxometalate of general formula $[XM^1M^2_{11}O_{40}]^{n-}$, wherein

X denotes P, Si, Ge, or Al;
$M^1$ denotes Mo, V, Ti, Nb;
$M^2$ denotes W or Mo;
n denotes 2-6.

According to a further embodiment of the invention the delivery molecule as described herein comprises a polyoxometalate of general formula $[XM^1M^2_{11}O_{39}(L)]^{n-}$, wherein

X denotes P, Si, or Ge
$M^1$ denotes Mo, V, Fe, Ga, In, Ni, Co, Cr, or Al;
$M^2$ denotes W or Mo;
L denotes a terminal ligand coordinated to $M^1$: $O^{2-}$, $H_2O$;
n denotes 3, 4, or 5.

A further embodiment relates to the delivery molecule as described herein, wherein the polyoxometalate is of formula $[X(OH)_6M_6O_{18}]^{3-}$, wherein

X denotes Al, Ga, Cr, or Fe;
M denotes Mo, or W.

A further embodiment relates to the delivery molecule as described herein, wherein the polyoxometalate is of formula $[PVW_{11}O_{40}]^{4-}$, or $[SiMoW_{11}O_{40}]^{4-}$.

According to one embodiment of the invention, the polyoxometalate is stable at a pH of up to 7.5.

According to one embodiment of the invention, the cargo molecule is a hydrophilic molecule or an organic compound.

According to one embodiment of the invention, the cargo molecule is a hydrophilic peptide or protein.

According to another embodiment of the invention, the cargo molecule is a pharmaceutically active compound.

According to another embodiment of the invention, the pharmaceutically active compound may be a hydrophilic moiety or an organic compound.

A further embodiment of the invention relates to a pharmaceutically active compound used as cargo molecule as described herein. The pharmaceutically active compound may be a hydrophilic peptide or protein, an organic compound or inorganic compound.

A further embodiment relates to the delivery molecule as described herein, wherein said polyoxometalate carrier is attached to said cargo molecule through reversible non-covalent supramolecular interactions.

A further embodiment relates to the use of the delivery molecule as described herein in delivering a therapy to or removing a therapy from a site within a subject. The site within said subject may be a cell and its barrier is a cell membrane.

A further embodiment relates to *in vitro* method for delivering a cargo molecule to a cell, comprising the steps of

a. providing a cell, and
b. contacting said cell with a delivery molecule as described herein.

One embodiment of the invention relates to the delivery molecule as described herein as a medicament.

A further embodiment relates to the delivery molecule as described herein, for use in the treatment of a disease curable by a pharmaceutically active molecule.

Another embodiment of the invention relates to the delivery molecule as described herein, for use in the treatment of a disease curable by a pharmaceutically active molecule, or hydrophilic peptide or protein.

One embodiment of the invention relates to a pharmaceutical composition comprising the delivery molecule as described herein.

**Brief description of drawings**

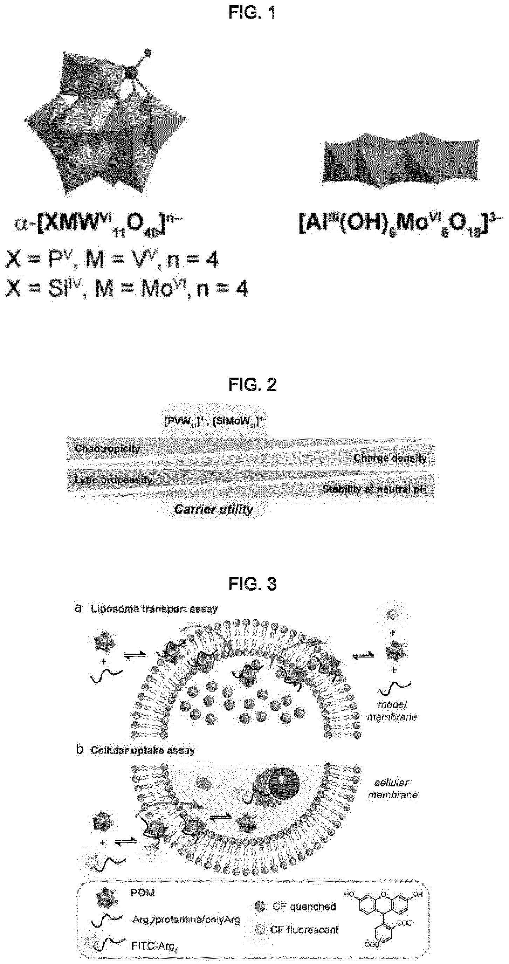

Fig. 1 POM archetypes. The Keggin (left) and the Anderson (right) archetype.

Fig. 2 Ordering of the POMs according to their chaotropicity (projected based on their charge density), their experimental pH stability, and expected membrane lytic propensity; the window of POMs with practical utility as membrane carriers is shown in yellow.

Fig. 3 (a) Schematic representation of the transport of impermeable oligoarginines facilitated by POMs in model membranes. (b) Cellular uptake of the labelled oligoarginine assisted by POMs.

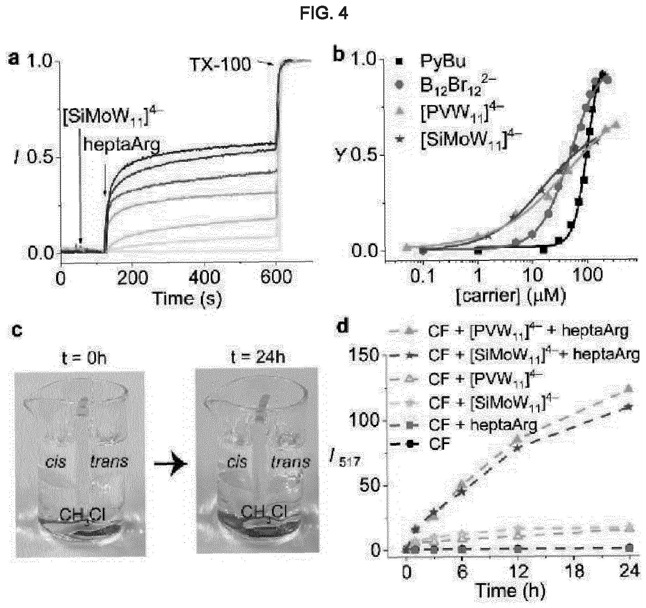

Fig. 4 (a) Changes in CF emission ($\lambda_{ex}$ = 492 nm, $\lambda_{em}$ = 517 nm) in EYPC⊃CF vesicles as a function of time during the addition of increasing concentrations of $[SiMoW_{11}]^{4-}$ (0-150 $\mu$M), heptaArg (10 $\mu$M), and TX100, for calibration. (b) Dose response curves for heptaArg transport for the active POMs and the corresponding Hill curve fits in comparison with the standards. (c) Pictures of the U-tube experiment (conducted with $[SiMoW_{11}]^{4-}$) at the opening (t = 0) and the closing (t = 24 h) of the experiment. (d) CF fluorescence measured in the trans phase of the U-tube. In the U-tube experiments, CF, POM, and heptaArg were fixed as 100 $\mu$M, 50 $\mu$M, and 10 $\mu$M).

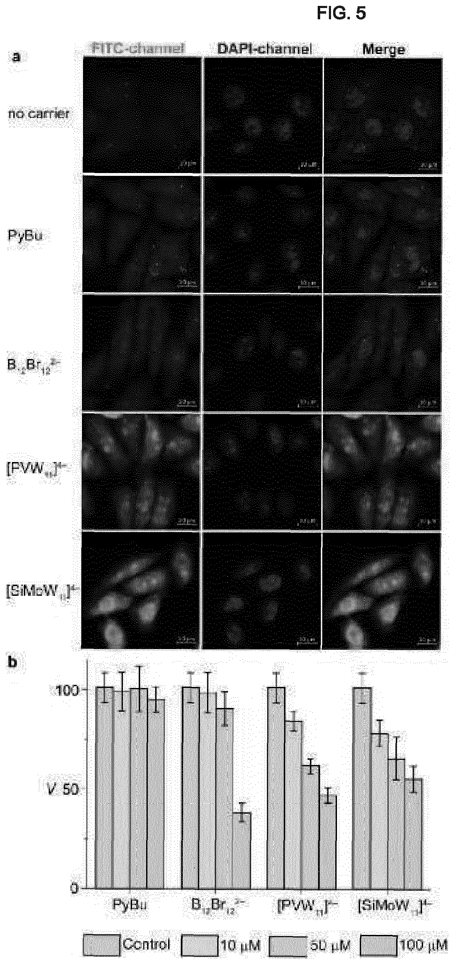

Fig. 5 (a) Confocal micrographs of $^{FITC}$Arg8 uptake (4 $\mu$M) alone (no carrier) and promoted by different carriers (5 $\mu$M)

in CHO-K1 cells. Inset scale bars: 10 $\mu$m. Representative images of two biological replicates. (b) MTT viability assay of CHO-K1 at different carrier concentrations (0 $\mu$M, 10 $\mu$M, 50 $\mu$M, and 100 $\mu$M). Error bars refer to standard deviation (n = 3).

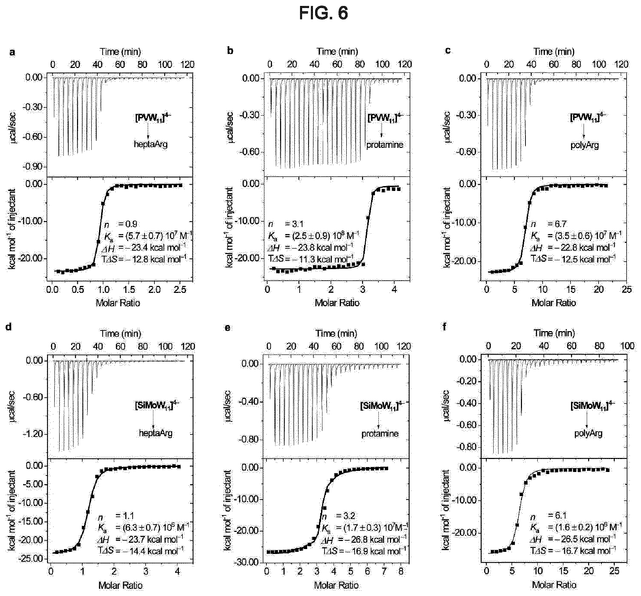

Fig. 6 Microcalorimetric titrations in 10 mM Mes, pH 5.5: Raw ITC data (top) for the sequential injections of POM solution into an oligoarginine solution, and apparent reaction heats (bottom) obtained from the integration of the calorimetric traces. POM/oligoarginine concentrations in $\mu$M: a, 100/8; b, 100/5; c, 100/1; d, 200/10; e, 100/3; and f, 100/0.9. Error data is 5 % for the n values and $\pm$ 0.5 kcal mol$^{-1}$ for $\Delta H$ and $T\Delta S$ (duplicate measurements). Note that none of these interactions lead to any precipitation or aggregates, confirmed by the absence of any detectable signal by DLS.

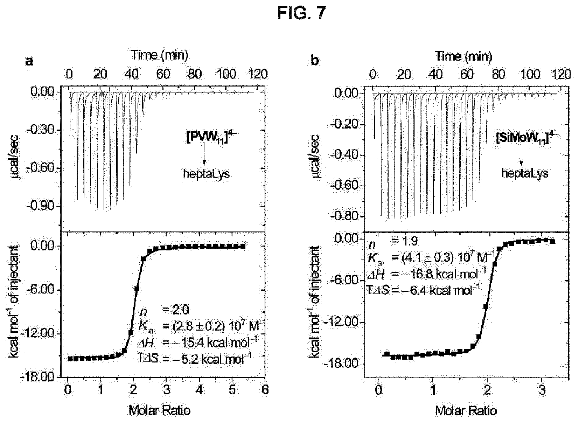

Fig. 7 Microcalorimetric titrations in 10 mM Mes, pH 5.5: Raw ITC data (top) for the sequential injections of POM solution into a heptalysine solution, and apparent reaction heats (bottom) obtained from the integration of the calorimetric traces. POM/heptalysine concentrations in $\mu$M: a, 200/8 and b, 150/10. Error data is 5 % for the n values and $\pm$ 0.5 kcal mol$^{-1}$ for $\Delta H$ and $T\Delta S$ (duplicate measurements). Note that these interactions did not lead to any precipitation or aggregates, confirmed by the absence of any detectable signal by DLS.

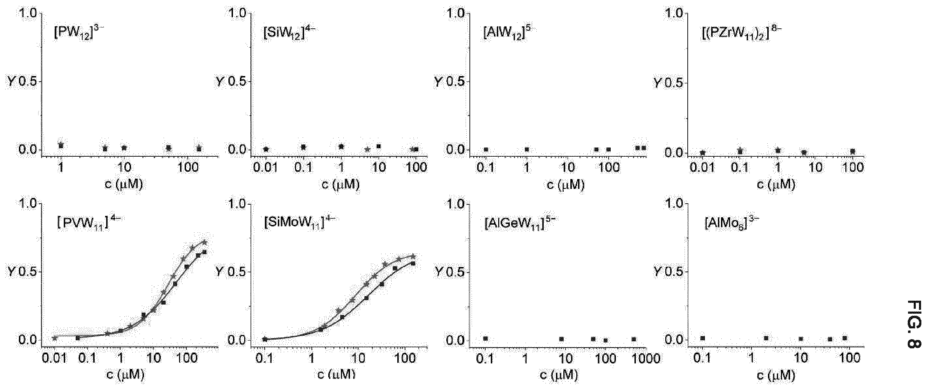

Fig. 8 Transport activity, $Y$, for heptaarginine in (10 $\mu$M) EYPC$\supset$CF liposomes as a function of the concentration of the different evaluated POMs. Solid lines correspond to the resulting fit with the Hill equation. Blue squares are data obtained at neutral pH, and red stars data obtained at acidic pH.

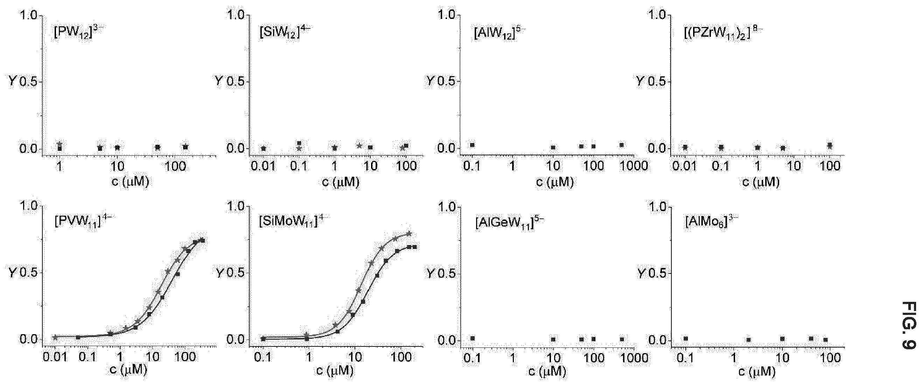

Fig. 9 Transport activity, $Y$, for protamine (1 $\mu$M) in EYPC$\supset$CF liposomes as a function of the concentration of the different evaluated POMs. Solid lines correspond to the resulting fit with the Hill equation. Blue squares are data obtained at neutral pH, and red stars data obtained at acidic pH.

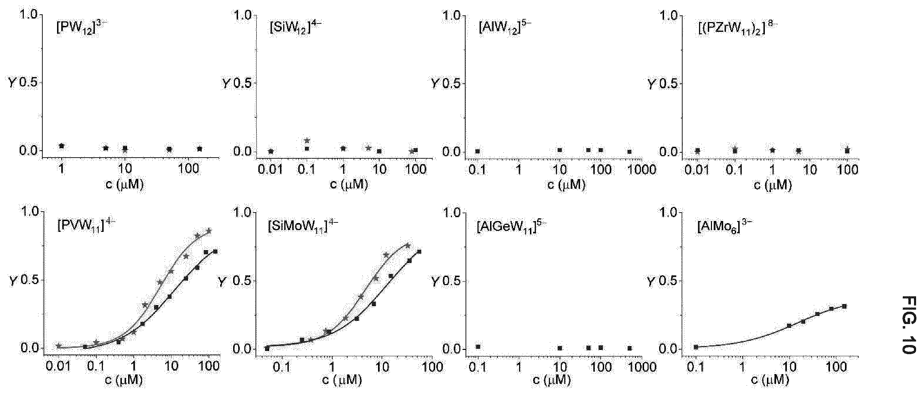

Fig. 10 Transport activity, Y, for polyarginine (0.1 $\mu$M) in EYPC$\supset$CF liposomes as a function of the concentration of the different evaluated POMs. Solid lines correspond to the resulting fit with the Hill equation. Blue squares are data obtained at neutral pH, and red stars data obtained at acidic pH.

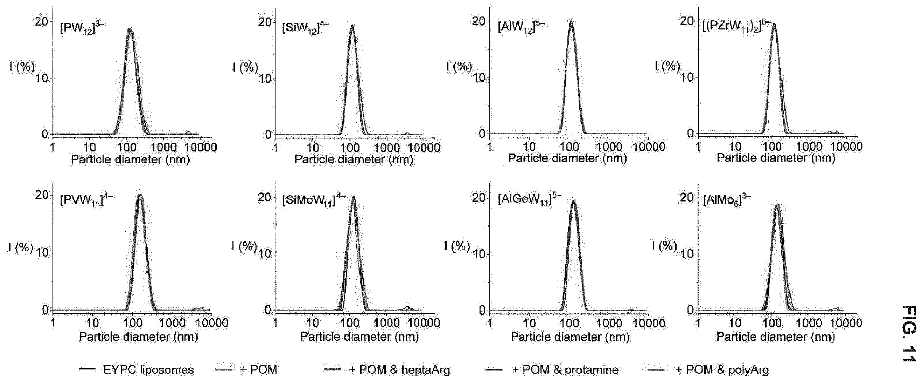

Fig. 11 Size distribution (by DLS) of EYPC$\supset$CF liposomes before (black trace), and after the addition of POM alone (100 $\mu$M, green trace), and in presence of heptaarginine (10 $\mu$M, red trace), protamine (1 $\mu$M, blue trace), or polyarginine (0.1 $\mu$M, purple trace). Higher $[PW_{12}]^{3-}$ and $[SiW_{12}]^{4-}$ lyse the vesicles.

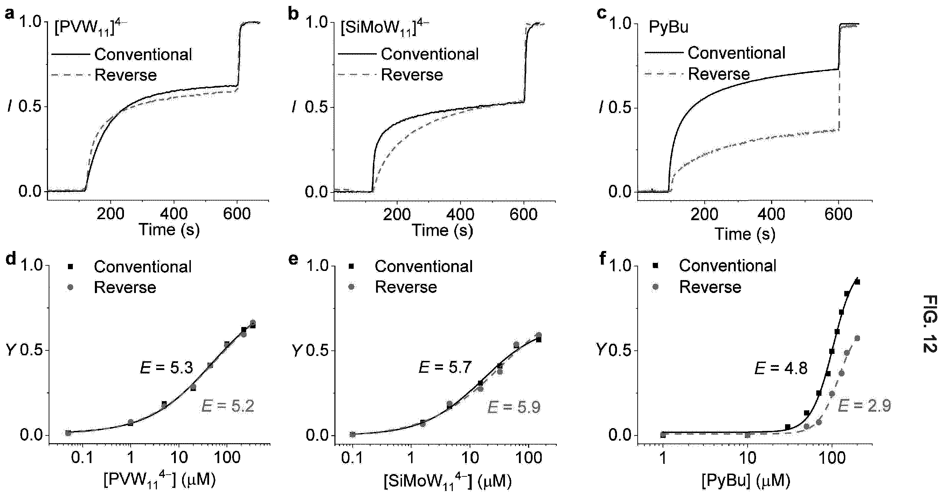

Fig. 12 Transport kinetics (upper panels) and dose response curves (bottom panels) for the transport of heptaarginine (10 $\mu$M) mediated by the superchaotropic POM carriers, $[PVW_{11}]^{4-}$ and $[SiMoW_{11}]^{4-}$, vs pyrenebutyrate (PyBu) in dependence of carrier-cargo sequence of addition. a, 200 $\mu$M $[PVW_{11}]^{4-}$. b, 60 $\mu$M $[SiMoW_{11}]^{4-}$. c, 120 $\mu$M PyBu.

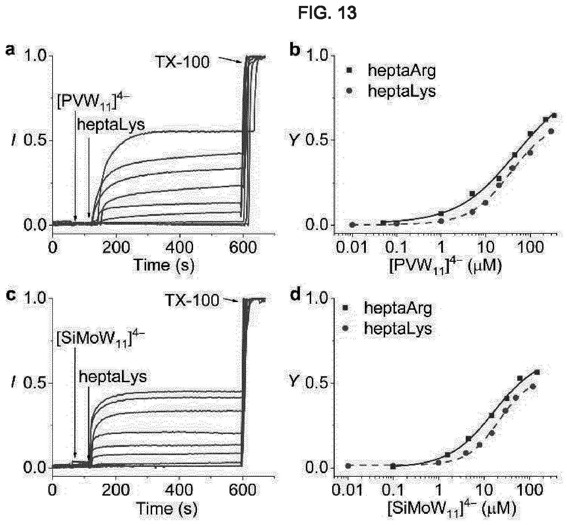

Fig. 13 Successful transport of the less basic lysine derivative with $[PVW_{11}]^{4-}$ (a) and $[SiMoW_{11}]^{4-}$ (c). Comparable transport of heptalysine (circles, dash purple line) and heptaarginine (10$\mu$M) (squares, solid blue line) activated by $[PVW_{11}]^{4-}$ (b) and $[SiMoW_{11}]^{4-}$ (d).

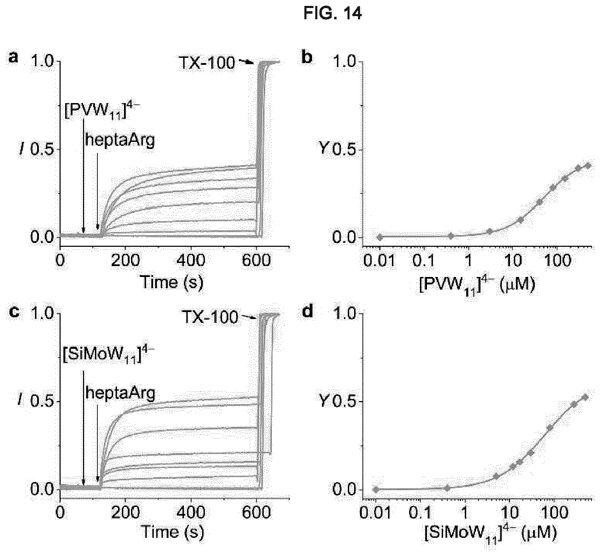

Fig. 14 Heptaarginine transport through anionic vesicles (DMPE/DPPG/CHOL, 1/2/1 molar ratio) facilitated by $[PVW_{11}]^{4-}$ (a) and $[SiMoW_{11}]^{4-}$ (c). Transport activity as function of $[PVW_{11}]^{4-}$ (b) and $[SiMoW_{11}]^{4-}$ (d) concentration, solid lines correspond to the resulting fit with the Hill equation.

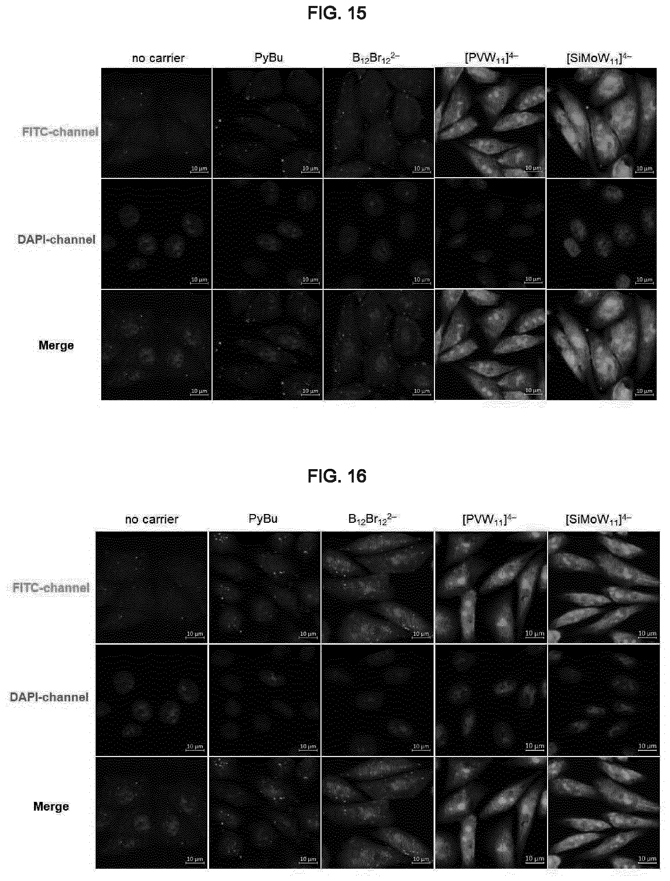

Fig. 15 Confocal fluorescence microscopy images of $^{FITC}Arg_8$ uptake (4 $\mu$M) alone (no carrier) and promoted by the different carriers (10 $\mu$M) in CHO-K1 cells. Inset scale bars: 10 $\mu$m. Representative images of two biological replicates.

Fig. 16 Confocal fluorescence microscopy images of $^{FITC}Arg_8$ uptake (4 $\mu$M) alone (no carrier) and promoted by the different carriers (50 $\mu$M) in CHO-K1 cells. Inset scale bars: 10 $\mu$m. Representative images of two biological replicates.

## Description of Embodiments

The present invention provides a delivery molecule comprising a polyoxometalate as carrier molecule and a cargo molecule selected from the group consisting of a hydrophilic moiety, an organic moiety, and an inorganic moiety.

As used herein, a "delivery molecule" refers to a molecule which comprises a carrier molecule and a cargo molecule, enabling the transport of the latter into a compartment enclosed by a chemical and/or biological barrier, and finally releasing said cargo molecule into said compartment.

As used herein, a "carrier molecule" refers to any molecule which is able to contacting a second molecule i.e., a cargo molecule, enabling the transport of the latter in to a compartment enclosed by a chemical and/or biological barrier, and finally releasing said substance into said compartment.

As used herein, a "cargo molecule" refers to any substance that is in contact with a carrier molecule and transported by said carrier molecule into a compartment enclosed by a chemical and/or biological barrier.

The term "linker-free" or "without a linker" as used herein refers to the absence of any intermediate compound which may connecting the carrier molecule and the cargo molecule via covalent and/or non-covalent interactions.

The term "hydrophilic moiety" as used herein refers to a structure that can dissolve in polar solutions (e.g., water). Usually, hydrophilic substances are polar in nature. Hydrophilic substances tend to readily dissolve in water or polar solvents.

Hydrophilic molecules or hydrophilic moieties are predominantly polar compounds that have ionic groups. The polar nature of these hydrophilic molecules or hydrophilic moieties enables them to readily absorb water or polar solvents and eventually get dissolved in polar solvents like water. Being a polar protic solvent, water is capable of forming a hydrogen bond (-H-OH-). Hydrophilic molecules or hydrophilic moieties are polar in nature and easily form a hydrogen bond(s) with water thereby getting dissolved in water. Notably, these interactions between the hydrophilic molecule or hydrophilic moiety and water are thermodynamically favored. In general, hydrophilic substances can easily form hydrogen bonds with polar solvents like water, and alcohol. The polarity of a moiety usually defines its hydrophilicity. Hydrophilic molecules usually cannot penetrate a lipid biolayer and cell membrane themselves.

The hydrophilic moiety according to the invention may be a peptide or protein having hydrophilic properties.

polyoxometalate (POM) refers to a polyatomic ion, usually an anion, that consists of three or more transition metal oxyanions linked together by shared oxygen atoms to form closed 3-dimensional chemical structures.

In one embodiment of this invention the metal atoms of the POM may be selected from the elements in groups 4 to 10, or group 13 of the latest released periodic table of the elements by International Union of Pure and Applied Chemistry (IUPAC), hereafter referred to as periodic table. The metal atom may be selected from the group consisting of titanium, chromium, vanadium, niobium, molybdenum, tungsten, iron, cobalt, nickel, gallium, and indium, or from the group consisting of titanium, vanadium, niobium, molybdenum, and tungsten, or from the group consisting of molybdenum, vanadium, iron, gallium, indium, nickel, cobalt, chromium, and tungsten. According to one embodiment of the invention, the metal ion is selected from the group consisting of vanadium, molybdenum, and tungsten.

According to one embodiment, the main group oxyanion of the POM may be formed with any of the elements in groups 14, or 15 of the periodic table, such as for example, from the group consisting of phosphor, silicon, and germanium. The hetero-ion of the POM may be selected from the group consisting of phosphate, silicate, and germanate. The ligand of the POM may be selected from the group of molecular oxygen and water.

The charge of the POM may be -6 to -2, or -5 to -3, or -4 to -3.

The term "supramolecular interaction" as used herein refers to an intermolecular non-covalent interaction between two molecules. These forces may include hydrogen bonding, metal coordination, hydrophobic forces, van der Waals forces, pi-pi interactions, or electrostatic effects, or combinations thereof. Interaction. Examples of supramolecular interactions include hydrogen bonding, hydrophobic interaction or hydrophobic bonding, electrostatic interaction or ionic bonding, salt bridging, and coordinate bonding.

As used herein, the term "pharmaceutically active compound" refers to any ingredient that provides biologically active or other direct effect in the diagnosis, cure, mitigation, treatment, or prevention of disease or to affect the structure or any function of the body of humans or animals.

Any pharmaceutically active compound which can be connected to the carrier molecule may be used according to the invention. Suitable pharmaceutically active compounds are for example, hydrophilic peptides or proteins such as phalloidin, pancuronium, vitamin $B_1$, cartilage derived retinoic acid sensitive protein (CD-RAP), bone morphogenetic protein (BMP), or kanamycin A. The pharmaceutical active compound may be an organic compound or even an inorganic compound such as cis-platin.

As used herein, the term "site" refers to a closed compartment which is enclosed by a chemical and/or biological barrier, e.g., to a cell enclosed by a plasma membrane, or to two phases separated by a chemical layer.

As used herein, a "superchaotropic effect" refers to a most extreme case of the chaotropic effect, which refers to the propensity of large anions to associate with hydrophobic and neutral polar phases, contrasting the hydrophobic effect as a distinct assembly motif in chemistry. Superchaotropic ions are characterized by enthalpically driven processes with negative entropic contributions. These superchaotropic anions would be introduced in the extended Hofmeister Serie between chaotropic and hydrophobic ions.

## Examples

The Examples which follow are set forth to aid in the understanding of the invention but are not intended to, and should not be construed to, limit the scope of the invention in any way. The Examples do not include detailed descriptions of conventional methods. Such methods are well known to those of ordinary skill in the art.

### Synthesis

All reagents and chemicals were of high-purity grade and were used as purchased without further purification. Carboxyfluorescein (CF), cholesterol, CHO-K1, dulbecco's phosphate buffered saline (DPBS), 1,2-dimyristoyl-sn-glycero-3-phosphoethanolamine (DMPE), 1,2-dipalmitoyl-sn-glycero-3-phospho-(1'-rac-glycerol) (DPPG), egg yolk phosphatidylcholine (EYPC), Hank's balanced salt solution, nutrient mixture F-12 Ham, protamine, pyrenebutyrate, were from sigma-Aldrich (Steinheim, Germany). Resazurin was from TCI (Eschborn, Germany). $Trp\text{-}Arg_7$ and $Trp\text{-}Lys_7$ were

custom-made by GeneCust (Boynes, France) in > 95 % purity as confirmed by HPLC and MS by the supplier. Peptide stock solutions were prepared in water and their concentration was determined by using the extinction coefficient of tryptophan at 280 nm ($\varepsilon$ = 5540 cm$^{-1}$ M$^{-1}$). FITC-Arg$_8$ was from GL Biochem (Shangai, China) Ltd, and its concentration was determined by using the extinction coefficient of 5-FITC at 491 nm ($\varepsilon$ = 73000 cm$^{-1}$ M$^{-1}$).

$Na_3[\alpha\text{-}P^VW^{VI}_{12}O_{40}] \cdot nH_2O$ ($[PW_{12}]^{3-}$ for $[\alpha\text{-}P^VW^{VI}_{12}O_{40}]^{3-}$) was purchased from Sigma-Aldrich.
$K_4[\alpha\text{-}Si^{IV}W^{VI}_{12}O_{40}] \cdot 14H_2O$ ($[SiW_{12}]^{4-}$ for $[\alpha\text{-}Si^{IV}W^{VI}_{12}O_{40}]^{4-}$) [3],
$Na_5[\alpha\text{-}Al^{III}W^{VI}_{12}O_{40}] \cdot 12H_2O$ ($[AlW_{12}]^{5-}$ for $[\alpha\text{-}Al^{III}W^{VI}_{12}O_{40}]^{5-}$) [4],
$(C_4H_{12}N)_4[\alpha\text{-}HAl^{III}Ge^{IV}W^{VI}_{11}O_{39}(H_2O)] \cdot 11H_2O$ ($[GeAlW_{11}]^{5-}$ for $[\alpha\text{-}Al^{III}Ge^{IV}W^{VI}_{11}O_{39}(H_2O)]^{5-}$) [5],
$K_4[\alpha\text{-}P^VV^VW^{VI}_{11}O_{40}] \cdot 4H_2O$ ($[PVW_{11}]^{4-}$ for $[\alpha\text{-}P^VV^VW^{VI}_{11}O_{40}]^{4-}$) [6],
$K_4[\alpha\text{-}Si^{IV}Mo^{VI}W^{VI}_{11}O_{40}] \cdot 24H_2O$ ($[SiMoW_{11}]^{4-}$ for $[\alpha\text{-}Si^{IV}Mo^{VI}W^{VI}_{11}O_{40}]^{4-}$) [7], $(Et_2NH_2)_8[\{\alpha\text{-}P^VW^{VI}_{11}O_{39}Zr^{IV}(\mu\text{-}OH)(H_2O)\}_2] \cdot 7H_2O$ ($[(PZrW_{11})_2]^{8-}$ for $[\{\alpha\text{-}P^VW^{VI}_{11}O_{39}Zr^{IV}(\mu\text{-}OH)(H_2O)\}_2]^{8-}$) [8],
$Na_3[Al^{III}(OH)_6Mo^{VI}_6O_{18}] \cdot 2H_2O$ ($[AlMo_6]^{3-}$ for $[Al^{III}(OH)_6Mo^{VI}_6O_{18}]^{3-}$) [9]

were prepared according to literature procedures. All salts were characterized in solution by NMR spectroscopy and in the solid-state using infrared (IR) spectroscopy and thermogravimetric analysis (TGA). IR bands (cm$^{-1}$) for the synthesized compounds: $[PW_{12}]^{3-}$ (1074, 971, 888, 769, 574, 511); $[PVW_{11}]^{4-}$ (1100, 1074, 1058, 972, 876, 760, 596, 515, 473, 428); $[(PZrW_{11})_2]^{8-}$ (1099, 1053, 956, 883, 767, 698, 656, 594, 513, 487, 408); $[SiW_{12}]^{4-}$ (1016, 970, 912, 881, 748, 521, 476); $[SiMoW_{11}]^{4-}$ (1016, 974, 908, 874, 729, 553, 528, 474, 416); $[AlW_{12}]^{5-}$ (962, 953, 904, 878, 795, 746, 530, 468); $[GeAlW_{11}]^{5-}$ (950, 874, 820, 764, 677, 517, 447); $[AlMo_6]^{3-}$ (943, 902, 867, 797, 723, 626, 611, 568, 509, 482, 447). The mass-loss for $[PW_{12}]^{3-}$ is 4.7 % that corresponds to 7 $H_2O$; for $[PVW_{11}]^{4-}$ is 2.5 % that corresponds to 4 $H_2O$; for $[(PZrW_{11})_2]^{8-}$ is 12.1 % that corresponds to 9 $H_2O$ and 8 $Et_2NH_2$; for $[SiMoW_{11}]^{4-}$ is 12.8 % that corresponds to 24 $H_2O$; for $[AlW_{12}]^{5-}$ is 6.8 % that corresponds to 12 $H_2O$; for $[GeAlW_{11}]^{5-}$ is 15.7 % that corresponds to 12 $H_2O$ and 4 $C_4H_{12}N$; for $[AlMo_6]^{3-}$ is 8.9 % that corresponds to 5 $H_2O$.

*Attenuated total reflection Fourier-transform Infrared Spectroscopy*

All FTIR spectra were recorded on a Broker Vertex 70 IR Spectrometer equipped with a single-reflection diamond-ATR unit. Frequencies are given in cm$^{-1}$, intensities denoted as w = weak, m = medium, s = strong, br = broad.

*Elemental analysis*

Elemental microanalysis of C/H/N/O contents was performed by Mikroanalytisches Laboratorium (University Vienna, Faculty of Chemistry). An EA 3000 (Eurovector) was used for C/H/N/S-analysis. O-determination was performed by high temperature digestion using the HT 1500 (Hekatech, Germany) pyrolysis system in combination with the EA 3000 system.

*Thermogravimetric analysis (TGA)*

*TGA* was performed on a Mettler SDTA851e Thermogravimetric Analyzer under $N_2$ flow with a heating rate of 5 K min$^{-1}$ in the region 298-873 K.

*Isothermal Titration Calorimetry (ITC)*

All experiments were performed with a VP-ITC MicroCalorimeter from MicroCal, Int,. at atmospheric pressure and 298.15 K. Solutions were degassed and thermostatic prior the titration experiments in a ThermoVac accessory. A constant volume of POM ($[PVW_{11}]^{4-}$ or $[SiMoW_{11}]^{4-}$) (10 $\mu$L per injection) was injected into the oligopeptide solution (heptaArg, protamine, polyArg or heptaLys) in 10 mM Mes, pH 5.5 to determine the apparent binding affinity of $[PVW_{11}]^{4-}$ and $[SiMoW_{11}]^{4-}$ with the oligopeptides. Dilution heats were determined by titration of the POM into buffer and subtracted from the reaction heat. The neat reaction heat was fitted with Origin 7.0 software by using the *"one-set-of-sites"* model to obtain the complex stability constant (Ka) and molar reaction enthalpy ($\Delta H°$). The free energy ($\Delta G°$) and entropy changes ($\Delta S°$) were obtained according to the relation: $\Delta G° = - R\, T\ln K_a = \Delta H° - T\Delta S°$.

*Vesicle preparation*

*Zwitterionic vesicles in neutral media:* A thin lipid film was prepared by evaporating a lipid solution of EYPC (25 mg) in $CHCl_3$ (1 mL) with a stream of nitrogen and then dried *in vacuo* overnight. The dry film was rehydrated with 1 mL buffer (50 mM CF, 10 mM Hepes, pH 7.4 or 50 mM CF, 10 mM Tris, pH 7.4) for 30 min at room temperature, subjected to freeze-thaw cycles (7 times) and extrusions (15 times) through a polycarbonate membrane (pore size 100 nm). Extravesicular components were removed by size exclusion chromatography (NAP-25 column Sephadex G-25 DNA grade) with 10 mM Hepes, 107 mM NaCl, pH 7.4 or 10 mM Hepes, 107 mM NaCl, pH 7.4. Phospholipid concentration was calculated by the Stewart assay.

*Zwitterionic vesicles in acidic media:* Acidic vesicles were prepared analogously but the rehydration and elution buffer were 50 mM CF, 10 mM Mes, pH 5.5 and 10 mM Mes, 107 mM NaCl, pH 5.5, respectively.

*Anionic vesicles in neutral media:* Anionic vesicles were prepared analogously except for the lipid composition, DMPE/DPPG/CHOL (4.4/10.4/2.6 mg, 1/2/1 molar ratio) in a 1:1 mixture of $CHCl_3$ and MeOH (1mL), the hydration (60 mins) and extrusion were performed at 55 °C, and the employed buffers were 100 mM CF, 10 mM Tris, pH 7.4 and 10 mM Tris, 140 mM NaCl, pH 7.4 for rehydration and elution, respectively.

*Transport experiments*

Vesicles stock solutions (5-10 μL) were diluted with the corresponding buffer in a disposable plastic cuvette and gently stirred (total volume 2000 μL, final lipid concentration 13 μM). Carboxyfluorescein fluorescence was monitored at $\lambda_{em}$=517 nm ($\lambda_{ex}$=492 nm) as a function of time after addition of POMs at 60 s, analyte at 120 s and Triton X-100 (24 μL 1.2 % wt/vol) at 600 s to lyse the vesicles, for calibration. Fluorescence intensities were normalized to fractional emission as:

$$I(t) = \frac{I_t - I_0}{I_\infty - I_0} \qquad (1)$$

Where $I_0 = I_t$ before POM addition and $I_0 = I_\infty$ after lysis. For Hill analysis, $I_t$ before lysis was defined as transport activity, $Y$, and plotted against POM concentration, $c$, and fitted to the Hill equation (2), to afford the activity in absence of POM, $Y_0$, the maximal activity, $Y_{max}$, the concentration needed to achieve 50 % of maximal activity, $EC_{50}$, and the Hill coefficient, $n$.

$$Y = Y_0 + \frac{Y_{max} - Y_0}{1 + \left(\frac{EC_{50}}{c}\right)^n} \qquad (2)$$

*Activator efficiency*

In the transport measurements, where different carriers (POMs) are tested with the same cargo, the activator efficiency ($E$) is determined by their capacity to transport the impermeable cargo to the intravesicular region and is characterized by $Y_{max}$, its maximal activity, and $EC_{50}$, the effective activator concentration. An ideal carrier reaches high $Y_{max}$ at low $EC_{50}$. To count both factors, the activator efficiency is defined as:

$$E = Y_{max} \frac{pEC_{50}}{f} \qquad (3)$$

were $pEC_{50}$ is the negative logarithm of the $EC_{50}$, and fa scaling factor, which was set to 20.6 to calibrate the highest (previously known) activation efficiency, $E$, as 10.

*U-tube transport experiments*

The U-tubes were house-made, similar to those of Rebek [10] and Matile [11] consisting in a small beaker with a central glass barrier, separating the two aqueous layers, *cis* (sampling phase) and *trans* (receiving phase), but allowing an interface chloroform layer below the *cis* and *trans* phases. 3 mL $CHCl_3$ are placed into the U-tube and 1 mL of the *cis* (combinations of carrier (POM), cargo (heptaArg) and CF in buffer) and *trans* (1 mL buffer) phases were added. The organic phase was stirred at 700 rpm at room temperature. Aliquots (25 μL) from the *trans* phase were taken at different times, 400 μL of buffer were added and measured by fluorescence.

*Cell culture and confocal imaging*

For confocal microscopy experiments, CHO-K1 cells were seeded into 12-well plates (Eppendorf) at a density of $1\times10^5$ cells/well (per 1 mL) and incubated for 24 h in Ham's F-12 containing 10 % (v/v) fetal bovine serum at 37 °C in a 5 % $CO_2$. The cells were washed twice with PBS, and then incubated with POMs and FITC-$Arg_8$ (4 $\mu$M) for 1 h at 37 °C, cells were washed twice with Hank's Balanced Salt solution containing 100 $\mu$g/mL heparin, twice again with PBS and fixed with 1 mL cold 4 % paraformaldehyde for 20 min. The cells were rewashed twice with PBS and subsequently the nuclei were stained with 2 $\mu$g/mL DAPI for 10 min at room temperature before imaging. Cells were imaged by LSM 980 confocal laser scanning microscope. Images were processed with Zen 3.2 software (Zeiss).

*Cellular viability assay*

For MTT assays in presence of activators, CHO-K1 cells were incubated in Ham's F-12 medium containing 10 % fetal bovine serum and 1.0 % penicillin streptomycin at 37 °C in a 5 % $CO_2$ for 24 hours. CHO-K1 cells were dispersed in a 96-well flat bottom tissue culture treated plates (Eppendorf) at density of $5\times10^3$ cells/well (per 100 $\mu$L) and incubated in Ham's F-12 containing 10 % (v/v) fetal bovine serum at 37 °C in a 5 % $CO_2$ incubator for 12 h to allow cell adhesion. Then, cells were incubated with POM, $B_{12}Br_{12}^{2-}$, or PyBu (10 $\mu$M, 50 $\mu$M, 100 $\mu$M) for another 24 h. After this incubation time, 20 $\mu$L resazurin solution (0.15 mg/mL) was added to cells and incubated for another 3 h. Finally, the plates were read at $\lambda_{em} =$ 590 nm ($\lambda_{ex} =$ 560 nm) using a JASCO fluorometer FP-8500.

The cell viability was evaluated by the following equation:

$$Cells\ viability = \frac{FI_{sample} - FI_{blank}}{FI_{control} - FI_{blank}} \times 100\ \% \qquad\qquad (4)$$

Where, $FI_{sample}$ is the fluorescence intensity of cells incubated with different compounds, $FI_{blank}$ is the fluorescence intensity of medium, and $FI_{control}$ is the fluorescence intensity of cells in absence of any compound.

**Results and Discussion**

POMs of the globular Keggin and planar Anderson archetype and with varying charge status from -3 to -8 (Fig. 1) were selected. To offset the known $[XW_{12}O_{40}]^{n-}$ (X = hetero-ion) hydrolysis propensity in aqueous solution, most pronounced at neutral pH, more highly charged mixed-metal derivatives (Fig. 1,lefthand side) were also included. The latter are obtained by hydrolytic removal of one $W=O^{4+}$ unit from the Keggin structure and the subsequent incorporation of an addenda metal to fill the cavity of the monolacunary intermediate. In detail, the following POM anions were studied (as acid or sodium, potassium, or tetraalkylammonium salts), see Fig. 1: $[\alpha-P^VW^{VI}_{12}O_{40}]^{3-}$ ($[PW_{12}]^{3-}$), $[\alpha-Si^{IV}W^{VI}_{12}O_{40}]^{4-}$ ($[SiW_{12}]^{4-}$), $[\alpha-Al^{III}W^{VI}_{12}O_{40}]^{5-}$ ($[AlW_{12}]^{5-}$), $[\alpha-P^VV^VW^{VI}_{11}O_{40}]^{4-}$ ($[PVW_{11}]^{4-}$), $[\alpha-Si^{IV}Mo^{VI}W^{VI}_{11}O_{40}]^{4-}$ ($[SiMoW_{11}]^{4-}$), $[\alpha-HAl^{III}Ge^{IV}W^{VI}_{11}O_{39}(H_2O)]^{5-}$ ($[GeAlW_{11}]^{5-}$), $[\{\alpha-P^VW^{VI}_{11}O_{39}Zr^{IV}(\mu-OH)(H_2O)\}_2]^{8-}$ ($[(PZrW_{11})_2]^{8-}$), and $[Al^{III}(OH)_6Mo^{VI}_6O_{18}]^{3-}$ ($[AlMo_6]^{3-}$). First, their long-term (24 h and longer) stability in aqueous solution (slightly acidic and neutral) was investigated by applying the active NMR nuclei in their composition (see). The Keggin anions $[PW_{12}]^{3-}$ and $[SiW_{12}]^{4-}$ were found to be fully hydrolyzed at neutral or even slightly acidic pH to their monolacunary forms (Fig. S29-S31), but they were included as negative controls, to exclude potential carrier activity arising from degradation products. The other selected POMs were found to be stable at neutral pH (7.5), or only very slowly hydrolyzed, as summarized in Table 1.

The capability of POMs to act as membrane carriers was explored first in large unilamellar vesicles (LUVs), loaded with carboxyfluorescein (CF). We selected three representative cationic peptides, heptaarginine (heptaArg), heptalysine (heptaLys), protamine, and polyarginine (polyArg), as test cargos. Inside the LUVs, the CF dye was encapsulated at self-quenching concentrations. With a suitable carrier, heptaArg can be taken up into the vesicles, bind with entrapped CF, and shuttle back to the exterior, spreading the previously quenched CF, and thereby restoring its fluorescence (Fig. 3). In the time-resolved fluorescence experiments, to test whether a POM acts as molecular carrier, the CF emission was continuously monitored during the addition of the inorganic carrier ($t$ = 60 s) and after injection the impermeable heptaArg ($t$ = 120 s). At the end of the experiment ($t$ = 600 s), the surfactant Triton X-100 was added to release all the entrapped CF and allow normalization of the intensity data (Fig. 4a).

It should be noted that the addition of the POMs alone (without cargo) retained the stability of the liposomes in the studied concentration range (up to 100 $\mu$M, see Fig. 11), that is, neither an increase in CF fluorescence nor changes in size distribution as obtained by dynamic light scattering experiments (DLS) were observed, which would have signaled membrane rupture. These negative controls allowed us to proceed to the carrier experiments, with added cargo. Amongst the eight inorganic clusters evaluated, we found two hits: The two mixed metal Keggin POMs $[PVW_{11}]^{4-}$ and $[SiMoW_{11}]^{4-}$ triggered the desired increase in fluorescence characteristic for cargo transport. Interestingly, the planar Anderson $[AlMo_6]$

3-showed also incipient transport activity, but only for polyarginine as cargo, while other Anderson-type POMs were inactive.

Table 1 Stability of selected POMs in acidic and neutral pH and membrane transport parameters at pH 7.4.

| Carrier | Hydrolysis/Stability | | HeptaArg[b] | | | Protamine[c] | | | PolyArg[d] | | |
|---|---|---|---|---|---|---|---|---|---|---|---|
| | pH 5.5 [e] | pH 7.5 [f] | $Y_{max}$[g](%) | $EC_{50}$[h] ($\mu$M) | $E$[i] | $Y_{max}$(%) | $EC_{50}$ ($\mu$M) | $E$ | $Y_{max}$(%) | $EC_{50}$ ($\mu$M) | $E$ |
| $[PW_{12}]^{3-}$ | to $[P^VW^{VI}_{11}O_{39}]^{7-}$ | / | - - -[j,k] | | | | | | | | |
| $[SiW_{12}]^{4-}$ | / | to $[Si^VW^{VI}_8O_{39}]^{8-}$ | - - -[jilt] | | | | | | | | |
| $[PVW_{11}]^{4-}$ | stable | 20 % to $[P^VW^{VI}_{11}O_{39}]^{7-}$ | 84 | 49 | 5.3 | 88 | 41 | 5.9 | 88 | 12 | 8.2 |
| $[SiMoW_{11}]^{4-}$ | stable | stable | 66 | 17 | 5.7 | 72 | 19 | 6.0 | 94 | 13 | 8.6 |
| $[AlW_{12}]^{5-}$ | / | stable | - - -[j] | | | | | | | | |
| $[AlGeW_{11}]^{5-}$ | / | stable | - - -[j] | | | | | | | | |
| $[(PZrW_{11})_2]^{8-}$ | / | stable | - - -[j] | | | | | | | | |
| $[AlMo_6]^{3-}$ | / | stable | - - -[j] | | | | | | 33 | 11 | 3.1 |
| $B_{12}Br_{12}^{2-[m]}$ | stable | stable | 95 | 48 | 6.1 | 84 | 11 | 8.0 | 94 | 2.6 | 12 |
| PyBu⁻ | stable | stable | 100 | 100 | 4.8 | 77 | 39 | 5.3 | 91 | 12 | 8.5 |

[a] EYPC vesicles. [b] 10 $\mu$M heptaArg. [c] 1 $\mu$M protamine. [d] 0.1 $\mu$M polyArg. [e] 10 mM Mes, 107 mM NaCl, pH 5.5. [f] 10 mM Hepes, 107 mM NaCl, pH 7.4 or 10 mM Tris, 107 mM NaCl, pH 7.4. [g] Maximal activity; $\pm 2$ % error (SD). [h] Effective concentration to reach 50 % of $Y_{max}$; 5 % error (SD). Activation efficiency $E = Y_{max}$ (p$EC_{50}$/f), were p$EC_{50}$ is the negative logarithm of $EC_{50}$, and fa scaling factor set to 20.6 to allocate $E$ between 0 and 10; 10 % error. No detectable activity at pH 5.5 and 7.4. [k] Membrane disruption observed at strongly acidic pH. [l] Carrier activity observed at strongly acidic pH. [m] Measured by HPTS/DPX assay.

Table 2. Results of stability investigation by NMR spectroscopy.

| POM | Buffer/Media | $^{31}P$, $^{51}V$, $^{183}W$ or $^{27}Al$ shifts, ppm | POM anions with % for parent (active) anion based on signals integration |
|---|---|---|---|
| [PVW$_{11}$]$^{4-}$ | D$_2$O | **$^{31}P$:** -14.8; **$^{51}V$:** -555 | **[P$^V$V$^V$W$^{VI}_{11}$O$_{40}$]$^{4-}$** |
| | Mes pH 5.5 freshly prepared | **$^{31}P$:** -10.8 and -14.8 | [P$^V$W$^{VI}_{11}$O$_{39}$]$^{7-}$ (1 %) and [P$^V$V$^V$W$^{VI}_{11}$O$_{40}$]$^{4-}$ (99 %) |
| | Mes pH 5.5 after 1 day | **$^{31}P$:** -10.8 and -14.8 | [P$^V$W$^{VI}_{11}$O$_{39}$]$^{7-}$ (1 %) and **[P$^V$V$^V$W$^{VI}_{11}$O$_{40}$]$^{4-}$** (99 %) |
| | Mes pH 5.5 after 5 days | **$^{31}P$:** -10.8 and -14.8 | [P$^V$W$^{VI}_{11}$O$_{39}$]$^{7-}$ (1 %) and **[P$^V$V$^V$W$^{VI}_{11}$O$_{40}$]$^{4-}$** (99 %) |
| | Tris-HCl pH 7.5 freshly prepared | **$^{31}P$:** -11.4, -14.4 and -14.9 | [P$^V$W$^{VI}_{11}$O$_{39}$]$^{7-}$ and **[P$^V$V$^V$W$^{VI}_{11}$O$_{40}$]$^{4-}$** (81 %) |
| | Tris-HCl pH 7.5 after 1 day | **$^{31}P$:** -11.4, -14.4 and -14.9 | [P$^V$W$^{VI}_{11}$O$_{39}$]$^{7-}$ and **[P$^V$V$^V$W$^{VI}_{11}$O$_{40}$]$^{4-}$** (71 %) |
| | Tris-HCl pH 7.5 after 5 days | **$^{31}P$:** -11.4, -14.4 and -14.9 | [P$^V$W$^{VI}_{11}$O$_{39}$]$^{7-}$ and **[P$^V$V$^V$W$^{VI}_{11}$O$_{40}$]$^{4-}$** (68 %) |
| | Nutrient mixture F-12 Ham[a] freshly prepared | **$^{31}P$:** 0.4 (free phosphate from medium), -10.6, -10.7, -10.8, -11.1 and -14.9; **$^{51}V$:** -509.2, -558.8 | [P$^V$W$^{VI}_{11}$O$_{39}$]$^{7-}$ (coordinated to different alkali and earth alkali metals leading to different shifts) and **[P$^V$V$^V$W$^{VI}_{11}$O$_{40}$]$^{4-}$** (43 %) |
| | Nutrient mixture F-12 Ham[a] after incubation for 1 h at 37 °C | **$^{31}P$:** 0.4 (free phosphate from medium), -10.1, -10.8, -13.7, -14.4 and -14.9; **$^{51}V$:** -509.2, -523.1, -526.7, -554.2, -558.8, -559.7 | [P$^V$W$^{VI}_{11}$O$_{39}$]$^{7-}$ (coordinated to different alkali and earth alkali metals leading to different shifts) and **[P$^V$V$^V$W$^{VI}_{11}$O$_{40}$]$^{4-}$** (16 %) |
| [SiMoW$_{11}$]$^{4-}$ | Mes pH 5.5 | **$^{183}W$:** -99.9, -100.2, -102.4, -103.0, -103.5, -107.4 | **[Si$^{IV}$Mo$^{VI}$W$^{VI}_{11}$O$_{40}$]$^{4-}$** |
| | Tris-HCl pH 7.5 | **$^{183}W$:** -99.3, -99.6, -101.8, -102.8, -106.7 | **[Si$^{IV}$Mo$^{VI}$W$^{VI}_{11}$O$_{40}$]$^{4-}$** |
| | Nutrient mixture F-12 Ham after incubation for 1 h at 37 °C | **$^{183}W$:** -99.4, -99.7, -101.9, -102.5, -102.9, -106.9 | **[Si$^{IV}$Mo$^{VI}$W$^{VI}_{11}$O$_{40}$]$^{4-}$** |
| [AlMo$_6$]$^{3-}$ | Hepes pH 7.5 freshly prepared | **$^{27}Al$:** 16.3 | [Al$^{III}$(OH)$_6$Mo$^{VI}_6$O$_{18}$]$^{3-}$ |
| | Hepes pH 7.5 after 1 day | **$^{27}Al$:** 16.3 | [Al$^{III}$(OH)$_6$Mo$^{VI}_6$O$_{18}$]$^{3-}$ |
| [AlW$_{12}$]$^{5-}$ | Tris-HCl pH 7.5 freshly prepared | **$^{27}Al$:** 72.6 | [Al$^{III}$W$^{VI}_{12}$O$_{40}$]$^{5-}$ |
| | Tris-HCl pH 7.5 after 1 day | **$^{27}Al$:** 72.6 | [Al$^{III}$W$^{VI}_{12}$O$_{40}$]$^{5-}$ |
| [GeAlW$_{11}$]$^{5-}$ | Tris-HCl pH 7.5 freshly prepared | **$^{27}Al$:** 9.4 | [HAl$^{III}$Ge$^{IV}$W$^{VI}_{11}$O$_{39}$(H$_2$O)]$^{4-}$ |
| | Tris-HCl pH 7.5 after 1 day | **$^{27}Al$:** 9.4 | [HAl$^{III}$Ge$^{IV}$W$^{VI}_{11}$O$_{39}$(H$_2$O)]$^{4-}$ |

(continued)

| POM | Buffer/Media | $^{31}P$, $^{51}V$, $^{183}W$ or $^{27}Al$ shifts, ppm | POM anions with % for parent (active) anion based on signals integration |
|---|---|---|---|
| $[(PZrW_{11})_2]^{8-}$ | Hepes pH 7.5 freshly prepared | $^{31}P$: -13.7, -14.8, -14.9 | $[\{\alpha\text{-}PW_{11}O_{39}Zr(\mu\text{-}OH)(H_2O)\}_2]^{8-}\cdot$ (99 %) and $[Zr(\alpha\text{-}PW_{11}O_{39})_2]^{10-}$ |
| | Hepes pH 7.5 after 1 day | $^{31}P$: -13.7, -14.8, -14.9 | $[\{\alpha\text{-}PW_{11}O_{39}Zr(\mu\text{-}OH)(H_2O)\}_2]^{8-}\cdot$ (99 %) and $[Zr(\alpha\text{-}PW_{11}O_{39})_2]^{10-}$ |
| $[PW_{12}]^{3-}$ | NaOAc/HOAc pH 4 freshly prepared | $^{31}P$: -12.4, -13.1, - 13.7; -15.2 | $[P^V W^{VI}_{12}O_{40}]^{3-}$ (30 %) and intermediate anions |
| | NaOAc/HOAc pH 4 after 1 day | $^{31}P$: -12.4, -13.1, - 13.7; -15.2 | $[P^V W^{VI}_{12}O_{40}]^{3-}$ (28 %) and intermediate anions |
| | Mes pH 5.5 freshly prepared | $^{31}P$: -11.0, -12.4; 13.1, -13.7; -14.7 | $[P^V W^{VI}_{12}O_{40}]^{3-}$ (4 %), $[P^V W^{VI}_{11}O_{39}]^{7-}$ (61 %) and intermediate anions |
| | Mes pH 5.5 after 1 day | $^{31}P$: -11.0, -12.4; 13.1; -13.3; -13.9; -14.7 | $[P^V W^{VI}_{12}O_{40}]^{3-}$ (5 %), $[P^V W^{VI}_{11}O_{39}]^{7-}$ (61 %) and intermediate anions |
| $[SiW_{12}]^{4-}$ | Hepes pH 7.5 | $^{183}W$: -99.8; -100.8; - 114.5; -119.8; -127.8; -140.4; -174.6 | $[Si^{IV} W^{VI}_{12}O_{40}]^{4-}$ and $[Si^{IV} W^{VI} nO_{39}]^{8-}$ (high noise to signal ratio, correct integration of peak cannot be performed; but clearly $[Si^{IV} W^{VI}_{11}O_{39}]^{8-}$ is predominant) |

[a] According to the provider Sigma-Aldrich, Nutrient Mixture F-12 Ham for cell culturing contains sodium pyruvate (0.11 g/L), phenol red, L-glutamine, and does not contain $NaHCO_3$ and Hepes, for more details please see https://www.sigmaaldrich.com/AT/en/technical-documents/technical-article/cell-culture-and-cell-culture-analysis/ mammalian-cell-culture/f-12-ham.

Within the Keggin series, the membrane carrier potential appears to be related to the chaotropicity of the clusters, which is expected to decrease with increasing charge status, namely $[PW_{12}]^{3-} > [SiW_{12}]^{4-}$, $[PVW_{11}]^{4-}$, $[SiMoW_{11}]^{4-} > [AlW_{12}]^{5-}$, $[AlGeW_{11}]^{5-}$. When $[PW_{12}]^{3-}$ and $[SiW_{12}]^{4-}$ were kept at pH 1.2, already the addition of $[PW_{12}]^{3-}$ alone to the liposomes caused an increase in fluorescence, showing strong interaction and membrane disruption. $[SiW_{12}]^{4-}$ showed the desired cargo transport at acidic pH, without membrane disruption. However, above pH 4.5, $[PW_{12}]^{3-}$ and $[SiW_{12}]^{4-}$ undergo rapid hydrolysis to afford the highly charged products of hydrolysis $[P^V W^{VI}_{11}O_{39}]^{7-}$ and $[Si^V W^{VI}_8O_{39}]^{8-}$, displaying neither an interaction with the membrane nor affecting cargo transport (Fig. 8-10). Accordingly, they have no biologically relevant carrier utility at neutral pH, discouraging biological use. In contrast, the two Al-based clusters $[AlW_{12}]^{5-}$ and $[AlGeW_{11}]^{5-}$ are stable at neutral pH, but their higher charge appears to adversely affect their transport property. The same applies for the sandwich cluster $[(PZrW_{11})_2]^{8-}$, whose total charge appears to be too high. Accordingly, we concentrated on the two mixed-metal clusters $[PVW_{11}]^{4-}$ and $[SiMoW_{11}]^{4-}$, which showed the desired interplay of sufficiently low net charge (-4), good hydrolytic stability near neutral pH, efficient carrier activity, and broad cargo scope.

To characterize the transport capability of the new inorganic carriers, the resulting normalized fluorescence traces were plotted versus the POM concentration, generating dose-response curves (Fig. 4b, Fig. 8-10), which could be analyzed by Hill analysis. The resulting membrane transport parameters are the maximal activity ($Y_{max}$), the POM concentration that is required to achieve 50 % of maximal activity ($EC_{50}$), and the activator efficiency ($E$), which are compiled in Table 1. For the selected cargos, the two POM carriers $[PVW_{11}]^{4-}$ and $[SiMoW_{11}]^{4-}$ show comparable $E$ values to the standards in the field, pyrenebutyrate (PyBu), as amphiphilic carrier, and $B_{12}Br_{12}^{2-}$, as superchaotropic carrier (Table 1). And although the transport in the vesicles does not reach the same high final $Y_{max}$ levels, which results in a lowering of the $E$ values, the onset for transport activity occurs at much lower carrier concentrations, around 1 $\mu$M (Fig. 4). Accordingly, the required carrier concentrations are desirably low, e.g., the $EC_{50}$ value of $[SiMoW_{11}]^{4-}$ is about a factor of 3 lower (16.5 $\mu$M versus 48 $\mu$M) than that of the boron cluster. The efficacy of the clusters did not markedly decrease when the sequence of addition was reversed (Fig. 12). This reflects the reversibility of the involved supramolecular interactions, which is not observed, for example, for PyBu, where (irreversible) cluster cargo aggregation is known to interfere and significantly reduces its activity (Fig. 12). Another contrast to the amphiphilic PyBu is that the carrier activity of the POM carriers was found to be transferable to the less basic heptalysine (Fig. 13); in fact, the activity characteristics for oligoarginine and oligolysine transport were very similar (Table 3). Note also that the carrier activity of $[PVW_{11}]^{4-}$ and $[SiMoW_{11}]^{4-}$ in the model membranes was found to be comparable at pH 5.0 and pH 7.4 for all cargos listed in Table 1 and 3, which showed that the minor hydrolysis observed for the former on long time scales at neutral pH (ca. 20 % after 24 h) did not affect its carrier activity significantly.

Table 3 Membrane transport parameters[a] of active POMs at neutral[b] and acidic[c] pH.

| carrier | pH | Hydrolysis/ Stability | HeptaArg[d] | | | Protamine[e] | | | PolyArg[f] | | |
|---|---|---|---|---|---|---|---|---|---|---|---|
| | | | $Y_{max}$ (%)[g] | $EC_{50}$[h] (μM) | $E$[i] | $Y_{max}$ (%) | $EC_{50}$ (μM) | $E$ | $Y_{max}$ (%) | $EC_{50}$ (μM) | $E$ |
| $[PVW_{11}]^{4-}$ | 7.5 | 20 % hydrolyzed to $[P^VW^{VI}_{11}O_{39}]^{7-}$ | 8 | 49 | 5.3 | 88 | 41 | 5.9 | 88 | 12 | 8.2 |
| | 5.5 | stable | 79 | 28 | 5.9 | 79 | 19 | 6.6 | 91 | 5 | 10.1 |
| $[SiMoW_{11}]^{4-\,[k]}$ | 7.5 | stable | 66 | 17 | 5.7 | 72 | 19 | 6.0 | 94 | 13 | 8.6 |
| | 5.5 | stable | 66 | 9 | 6.6 | 80 | 14 | 7.3 | 86 | 5 | 9.7 |

[a] EYPC vesicles. [b] 10 mM Tris, 107 mM NaCl, pH 7.4. [c] 10 mM Mes, 107 mM NaCl, pH 5.5. [d] 10 μM heptaArg. [e]1 μM Protamine. [f] 0.1 μM polyArg.
[g] Maximal activity; $\pm$2 % error (SD). [h] Effective concentration to reach 50 % activity of $Y_{max}$; 5 % error (SD). [i] Activation efficiency $E = Y_{max} (pEC_{50}/f)$, were $pEC_{50}$ is the negative logarithm of the $EC_{50}$, and fa scaling factor set to 20.6 to allocate $E$ between 0 and 10, 10 % error.

To affect membrane transport, any carrier needs to display an affinity towards the cargo as well as towards the lipid bilayer. Accordingly, the interaction between $[PVW_{11}]^{4-}$ and $[SiMoW_{11}]^{4-}$ and the cargos was analyzed by isothermal titration calorimetry (ITC). These experiments showed strong supramolecular interactions (on the order of $10^7$ M$^{-1}$) with affinities governed by the chaotropic effect (enthalpically driven, interaction with the peptide backbones, Fig. 6-7 and Table 4) and Coulombic interactions (interaction with arginine side chains). The strong but reversible intermolecular interactions between the low-charged POMs and the cationic peptides established by ITC present an interesting finding. Previous ITC studies have focused on the binding of highly charged and therefore weakly chaotropic POMs (n < -6) where electrostatic interactions were implied as main driving force.

Table 4 Binding constants ($K_a$ in M$^{-1}$) and thermodinamic data (in kcal mol$^{-1}$) measured for the complex formation between the stable and active POMs and oligoarginines in 10 mM Mes, pH 5.5. [a]

| | HeptaArg | | | | Protamine | | | | PolyArg | | | |
|---|---|---|---|---|---|---|---|---|---|---|---|---|
| | n | $K_a$/ $10^7$ | ΔH | TΔS | n | $K_a$/ $10^7$ | ΔH | TΔS | n | $K_a$/ $10^7$ | ΔH | TΔS |
| $[PVW_{11}]^{4-}$ | 0.90 | 5.7 ± 0.7 | -23.4 | -12.8 | 3.1 | 25 ± 9 | -23.8 | -11.3 | 6.7 | 3.5 ± 0.6 | -22.8 | -12.5 |
| $[SiMOW_{11}]^{4-}$ | 1.10 | 0.6 ± 0.1 | -23.7 | -14.4 | 3.2 | 1.7 ± 0.3 | -26.8 | -16.9 | 6.1 | 1.6 ± 0.2 | -26.5 | -16.7 |

[a] Error data is 5 % for the n values and $\pm$ 0.5 kcal mol$^{-1}$ for ΔH and TΔS (duplicate measurements.

U-tube transport experiments (see Methods, Fig. 2c-d), confirmed that $[PVW_{11}]^{4-}$ and $[SiMoW_{11}]^{4-}$ act as "real" carriers that can seize and transport a hydrophilic cargo even through a hydrophobic bulk phase. In the U-tube experiments, the *trans* phase contained only buffer, and the *cis* phase is loaded with combinations of CF, carrier (POM) and cargo (heptaArg). Aliquots of the *trans* phase are taken and evaluated by fluorescence at different times. The *trans* phase is initially *(t*= 0) nonfluorescent, but the transport of CF from the *cis* phase through the chloroform barrier leads to a fluorescence signal for successful transport. Fig. 4d shows that there is no fluorescence in the absence of POM, although an inefficient transport of CF can be sensed when the POM is mixed only with CF. In the ternary mixture, however, as expected, highly efficient transport is observed. This is consistent with the transport of a POM•heptaArg•CF complex through the organic phase. Accordingly, the superchaotropic properties of POMs cannot only be used for biological membrane transport, but these properties should be transferable to artificial membranes and phase-transfer processes in general, as recently demonstrated for catalysis and dissolution. To mimic biological membranes, which contain anionic components, equivalent transport experiments were performed in anionic liposomes with the prototypal POM carriers, $[PVW_{11}]^{4-}$ and $[SiMoW_{11}]^{4-}$. These experiments showed that, despite the apparently opposing negative charges, both POMs retained their carrier capabilities (Fig. 14, Table 5), with only slightly reduced net efficiencies. This encouraged us to transfer the experiments to CHO-K1 cells, after we had confirmed a sufficient stability of the most promising POM carriers, $[PVW_{11}]^{4-}$ and $[SiMoW_{11}]^{4-}$, in the cellular nutrient mixture (F-12 Ham's medium).

Table 5 Membrane transport parameters of active POMs towards different analytes and different lipid composition.

| Lipid[a] | POM | Cargo[b] | $Y_{max}$ (%)[c] | $EC_{50}$[d] ($\mu$M) | $E$[e] |
|---|---|---|---|---|---|
| EYPC (zwitterionic) | $[PVW_{11}]^{4-}$ | heptaArg | 84 | 49 | 5.3 |
| | | heptaLys | 62 | 38 | 4.3 |
| | $[SiMoW_{11}]^{4-}$ | heptaArg | 66 | 17 | 5.7 |
| | | heptaLys | 54 | 22 | 4.4 |
| DMPE/DPPG/ CHOL (anionic) | $[PVW_{11}]^{4-}$ | heptaArg | 45 | 50 | 2.8 |
| | $[SiMoW_{11}]^{4-}$ | heptaArg | 61 | 60 | 3.6 |

[a] 13 $\mu$M phospholipids. [b] 10 $\mu$M cargo, [c] Maximal activity; $\pm 2$ % error (SD). [d] Effective concentration to reach 50 % activity of $Y_{max}$; 5 % error (SD). [e] Activation efficiency $E = Y_{max}$ ($pEC_{50}/f$), were $pEC_{50}$ is the negative logarithm of the $EC_{50}$, and fa scaling factor set to 20.6 to allocate $E$ between 0 and 10; 10 % error.

POMs are known to cause cellular damage, and their antimicrobial as well as anticancer effects rest on those. Nevertheless, we tried to define a "therapeutic window" in which the cytotoxic properties would be sufficiently low to allow their carrier activity to be investigated (Fig. 5b). Indeed, at concentrations of 10 $\mu$M and below, $[PVW_{11}]^{4-}$ and $[SiMoW_{11}]^{4-}$, the CHO-K1 cells retained about the 80 % viability after 24 h according to MTT assay (Fig. 5b), while the onset of carrier activity in the negatively charged model membranes fell in the same range (Fig. 14). Accordingly, the uptake of 5(6)-fluorescein isothiocyanate-labelled octaarginine ($^{FITC}Arg_8$) was evaluated by confocal fluorescence microscopy at 5 and 10 $\mu$M carrier concentration (Fig. 5a and Fig. 15). In the absence of carrier, the cells showed minor punctate fluorescence, in agreement with (undesirable) endosomal uptake and entrapment of $^{FITC}Arg_8$. In the presence of the POM carriers, strong diffuse fluorescence was observed in both the cytosol and in the nucleus (Fig. 5a). Successful experiments were also conducted at 50 $\mu$M (Fig. 16), when the incubation time was kept much shorter than the 24 h used in the MTT assay. Consequently, while the present cell-biological results are preliminary, and attempted in fixed cells, the transport activity of the POMs is transferable from model membranes to cells. In fact, while the carrier efficiency in the vesicles was comparable (Table 1), the $^{FITC}Arg_8$ cell staining results for both POM carriers visually exceeded those by the alternative amphiphilic and superchaotropic carriers, PyBu and $B_{12}Br_{12}^{2-}$.

**References**

[1] Zhang Z. et al., Biomaterials 2015, 65, 56-65
[2] Barba-Bon A. et al., Nature 2022, 603, 637- 642
[3] Téazéa, G. H. A. et al., in Inorg. Synth., Vol. 27 (Ed.: A. P. Ginsberg), John Wiley & Sons,Inc., 1990, 85-96
[4] Weinstock, I. A. et al., Am. Chem. Soc. 1999, 121, 4608-4617
[5] Weinstock, I. A. et al., J. Am. Chem. Soc. 1999, 121, 4608-4617
[6] Salvi G. et al., Proteins: Struct., Funct., Bioinf. 2005, 61, 492-499
[7] Assaf K. I. et al., Chem. Int. Ed. 2018, 57, 13968-13981
[8] Ly, H. G. T. et al., Dalton Trans. 2013, 42, 10929-10938.
[9] Manikumari, S. et al., Inorg. Chem. 2002, 41, 6953-6955.
[10] Rebek J. A. et al., J. Am. Chem. Soc. 1987, 109, 2432-2434
[11] Sakai N. et al., J. Am. Chem. Soc. 2003, 125, 14348-14356

**Claims**

1. A delivery molecule comprising a carrier molecule and a cargo molecule, wherein said carrier molecule is a polyoxometalate moiety which exhibits a charge status of-2 to -6 and wherein the cargo molecule is a hydrophilic moiety, an inorganic molecule, or an organic molecule.

2. The delivery molecule of claim 1, wherein the carrier molecule and the cargo molecule are linked to each other without a linker molecule.

3. The delivery molecule of claim 1 or 2, wherein the polyoxometalate moiety has the general formula $[XM^1M^2{}_{11}O_{40}]^{n-}$, wherein

X denotes P, Si, Ge, or Al;
$M^1$ denotes Mo, V, Ti, or Nb;
$M^2$ denotes W, or Mo;
n denotes 2-6, or
of general formula $[XM^1M^2{}_{11}O_{39}(L)]^{n-}$, wherein
X denotes P, Si, or Ge;
$M^1$ denotes Mo, V, Fe, Ga, In, Ni, Co, Cr, or Al;
$M^2$ denotes W, or Mo;
L is a terminal ligand and denotes $O^{2-}$ or $H_2O$ coordinated to $M^1$;
n denotes 3-5., or
of general formula $[X(OH)_6M_6O_{18}]^{3-}$, wherein
X denotes Al, Ga, Cr, or Fe; and
M denotes Mo, or W.

4. The delivery molecule of claim 3, wherein the polyoxometalate is of formula $[PVW_{11}O_{40}]^{4-}$, or $[SiMoW_{11}O_{40}]^{4-}$.

5. The delivery molecule of any one of claims 1 to 4, wherein said polyoxometalate is stable at a pH up to 7.5.

6. The delivery molecule of any one of claims 1 to 5, wherein said polyoxometalate moiety is attached to said cargo molecule through supramolecular interactions.

7. The delivery molecule of any one of claims 1 to 6, wherein said cargo molecule is a hydrophilic moiety, or a hydrophilic peptide or protein.

8. The delivery molecule of any one of claims 1 to 7, wherein said cargo molecule is a pharmaceutically active compound.

9. The delivery molecule of claim 8, wherein said pharmaceutically active compound is a pharmaceutically active hydrophilic peptide or protein, or an organic, or an inorganic compound.

10. The delivery molecule of any one of claims 1 to 9 for use in delivering a therapy to or removing a therapy from a site within a subject.

11. The delivery molecule for use according to claim 10, wherein said delivery molecule passes through the barrier of the site based on a superchaotropic effect and wherein the site within said subject is a cell and its barrier is a cell membrane.

12. An *in vitro* method for delivering a hydrophilic moiety to a cell comprising the steps of

(a) providing a cell, and
(b) contacting said cell with a delivery molecule according to any one of claims 1 to 9.

13. The delivery molecule according to any one of claims 1 to 9 as a medicament.

14. The delivery molecule according to any one of claims 1 to 9 for use in the treatment of a disease curable by a pharmaceutically active compound.

15. A pharmaceutical composition comprising the delivery molecule according to any one of claims 1 to 9.

## FIG. 1

$\alpha\text{-}[XMW^{VI}_{11}O_{40}]^{n-}$

$X = P^V, M = V^V, n = 4$

$X = Si^{IV}, M = Mo^{VI}, n = 4$

$[Al^{III}(OH)_6 Mo^{VI}_6 O_{18}]^{3-}$

## FIG. 2

$[PVW_{11}]^{4-}$, $[SiMoW_{11}]^{4-}$

Chaotropicity — Charge density

Lytic propensity — Stability at neutral pH

*Carrier utility*

## FIG. 3

a  Liposome transport assay

*model membrane*

b  Cellular uptake assay

*cellular membrane*

POM

Arg₇/protamine/polyArg

FITC-Arg₆

CF quenched

CF fluorescent

FIG. 4

FIG. 5

FIG. 6

FIG. 7

EP 4 483 904 A1

FIG. 8

FIG. 9

FIG. 10

FIG. 11

**a** $[PVW_{11}]^{4-}$
Conventional
Reverse
$I$
Time (s)

**b** $[SiMoW_{11}]^{4-}$
Conventional
Reverse
$I$
Time (s)

**c** PyBu
Conventional
Reverse
$I$
Time (s)

**d** ■ Conventional
● Reverse
$Y$
$E = 5.3$
$E = 5.2$
$[PVW_{11}^{4-}]$ ($\mu$M)

**e** ■ Conventional
● Reverse
$Y$
$E = 5.7$
$E = 5.9$
$[SiMoW_{11}^{4-}]$ ($\mu$M)

**f** ■ Conventional
● Reverse
$Y$
$E = 4.8$
$E = 2.9$
$[PyBu]$ ($\mu$M)

FIG. 12

EP 4 483 904 A1

FIG. 13

FIG. 14

## FIG. 15

## FIG. 16

Europäisches
Patentamt

European
Patent Office

Office européen
des brevets

# EUROPEAN SEARCH REPORT

Application Number

EP 23 18 2286

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALEKSANDAR BIJELIC ET AL: "Polyoxometalates as Potential Next-Generation Metallodrugs in the Combat Against Cancer", ANGEWANDTE CHEMIE INTERNATIONAL EDITION, VERLAG CHEMIE, HOBOKEN, USA, vol. 58, no. 10, 12 October 2018 (2018-10-12), pages 2980-2999, XP072091064, ISSN: 1433-7851, DOI: 10.1002/ANIE.201803868 | 1-3,5-15 | INV. A61K47/52 |
| A | * page 2984, right column to page 2991, left column. * | 4 | |
| X | GEISBERGER GEORG ET AL: "Trimethyl and carboxymethyl chitosan carriers for bio-active polymer-inorganic nanocomposites", CARBOHYDRATE POLYMERS, vol. 91, no. 1, 1 January 2013 (2013-01-01), pages 58-67, XP093110325, GB ISSN: 0144-8617, DOI: 10.1016/j.carbpol.2012.08.009 | 1-3,5-7, 10-15 | |
| A | * figures 1-2, page 62, paragraphs 3.7-3.8 * | 4 | |

TECHNICAL FIELDS SEARCHED (IPC)

A61K

The present search report has been drawn up for all claims

| Place of search | Date of completion of the search | Examiner |
|---|---|---|
| The Hague | 8 December 2023 | Burema, Shiri |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................

& : member of the same patent family, corresponding
document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011045296 A
- EP 1316318 A

**Non-patent literature cited in the description**

- **ZHANG Z. et al.** *Biomaterials*, 2015, vol. 65, 56-65
- **BARBA-BON A. et al.** *Nature*, 2022, vol. 603, 637-642
- **TÉAZÉA, G. H. A. et al.** Inorg. Synth.. John Wiley & Sons,Inc., 1990, vol. 27, 85-96
- **WEINSTOCK, I. A. et al.** *Am. Chem. Soc.*, 1999, vol. 121, 4608-4617
- **WEINSTOCK, I. A. et al.** *J. Am. Chem. Soc.*, 1999, vol. 121, 4608-4617
- **SALVI G. et al.** *Proteins: Struct., Funct., Bioinf.*, 2005, vol. 61, 492-499
- **ASSAF K. I. et al.** *Chem. Int. Ed.*, 2018, vol. 57, 13968-13981
- **LY, H. G. T. et al.** *Dalton Trans.*, 2013, vol. 42, 10929-10938
- **MANIKUMARI, S. et al.** *Inorg. Chem.*, 2002, vol. 41, 6953-6955
- **REBEK J. A. et al.** *J. Am. Chem. Soc.*, 1987, vol. 109, 2432-2434
- **SAKAI N. et al.** *J. Am. Chem. Soc.*, 2003, vol. 125, 14348-14356